(19) **Europäisches Patentamt / European Patent Office / Office européen des brevets**

(11) **EP 4 494 561 A1**

(12) **EUROPEAN PATENT APPLICATION**

(43) Date of publication:
**22.01.2025 Bulletin 2025/04**

(21) Application number: **24189619.0**

(22) Date of filing: **19.07.2024**

(51) International Patent Classification (IPC):
**A61B 5/367** (2021.01) **A61B 5/00** (2006.01)

(52) Cooperative Patent Classification (CPC):
**A61B 5/367; A61B 5/7221;** A61B 5/287;
A61B 5/339; A61B 5/743; A61B 2576/023

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.07.2023 US 202318225014**

(71) Applicant: **Biosense Webster (Israel) Ltd.**
**2066717 Yokneam (IL)**

(72) Inventors:
• **SEGEV, Meytal**
**2066717 Yokneam (IL)**
• **MASSARWA, Fady**
**2066717 Yokneam (IL)**
• **ZOABI, Akram**
**2066717 Yokneam (IL)**
• **ALTMAN, Sigal**
**2066717 Yokneam (IL)**

(74) Representative: **Carpmaels & Ransford LLP**
**One Southampton Row**
**London WC1B 5HA (GB)**

(54) **ESTIMATION AND VISUALIZATION OF ELECTROANATOMICAL (EA) ANALYSIS QUALITY OF AN EA MAP**

(57)    A method includes receiving an electroanatomical (EA) map of at least a portion of a cardiac chamber. A quality is scored of an EA analysis of the EA map. Based on the scoring, one or more regions are identified in the mapped portion as failing to meet a defined quality criterion. The one or more regions are indicted on an EA map to be displayed to a user.

```
┌─────────────────────────────────────────────────────┐
│  Receive EA map of a portion of a cardiac chamber    │─── 302
└─────────────────────────────────────────────────────┘
                         │
┌─────────────────────────────────────────────────────┐
│   Score the EA analysis quality at the EA mapped     │─── 304
│                     portion                           │
└─────────────────────────────────────────────────────┘
                         │
┌─────────────────────────────────────────────────────┐
│  Based on the score, estimate one or more regions    │─── 306
│  in the mapped portion where the EA analysis is       │
│                   insufficient                        │
└─────────────────────────────────────────────────────┘
                         │
┌─────────────────────────────────────────────────────┐
│     Indicate the one or more regions on the EA map   │─── 308
└─────────────────────────────────────────────────────┘
                         │
┌─────────────────────────────────────────────────────┐
│  Display the EA analysis quality indicated EA map    │─── 310
│                     to a user                         │
└─────────────────────────────────────────────────────┘
```

*FIG. 3*

EP 4 494 561 A1

## Description

### FIELD OF THE DISCLOSURE

This disclosure relates generally to the analysis of electroanatomical (EA) maps, and specifically to a system and method for visualization of analysis quality in EA maps.

### BACKGROUND OF THE DISCLOSURE

EA map visualization methods were previously considered in the patent literature. For example, U.S. Patent 11,478,182 , filed 07 Jan 2021, describes a method including receiving (i) a modeled surface of at least a portion of a heart and (ii) multiple EA values measured at multiple respective positions in the heart. Multiple regions are defined on the modeled surface and, for each region, a confidence level is estimated for the EA values whose positions fall in the region. The modeled surface is presented to a user, including (i) the EA values overlaid on the modeled surface, and (ii) the confidence level graphically visualized in each region of the modeled surface.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a catheter-based electroanatomical (EA) mapping and ablation system, in accordance with an example of the present disclosure;

Fig. 2A is an EA map of a left atrium, in accordance with an example of the present disclosure;

Fig. 2B is a shading mask to indicate regions of the EA map of Fig. 2A having insufficient EA analysis quality, in accordance with an example of the present disclosure;

Fig. 2C is a combined EA map that results from overlaying the mask of Fig. 2B over the EA map of Fig. 2A, in accordance with an example of the present disclosure; and

Fig. 3 is a flow chart that schematically illustrates a method of estimation and visualization of the EA analysis quality of an EA map, in accordance with an example of the present disclosure.

### DETAILED DESCRIPTION OF EXAMPLES

OVERVIEW

Electroanatomical (EA) mapping of a cardiac chamber of a patient suffering from arrhythmia demands the acquisition of a sufficient number of data points that adequately capture each chamber wall location and respective electrical information. A physician performing cardiac EA mapping aims to achieve an optimal diagnostic quality EA map of at least a portion of the cardiac chamber. The quality of the EA map depends, for example, on the stability of the arrhythmia and the ability to cover a sufficient portion of the chamber. EA quality is reflected in the output of dedicated algorithms that analyze EA properties based on an acquired set of data points.

Examples of an analysis performed by such EA algorithms include calculation of local conduction vectors (LCV) that are based on the strongest detected trend in local activation times (LAT), Coherent mapping analysis, and touch proximity index (TPI). These calculations will result in an output after spatial interpolation. Other examples include CARTO® points/"visitags" distance from surface, and data points density that if not accurate/sufficient may cause geomantic anatomical inaccuracies

As found by the inventors, EP quality of the above EA analysis algorithms may be insufficient or lacking in some regions of an EA map without the user being fully aware of this fact. For example, in LCV algorithm, if the algorithm cannot detect a trend in LAT values it will not present the vectors over the map. A lack of trend can be caused due to insufficient data points at a designated area or intramural signals that may present similar LAT values. Another example is coherent mapping where too excessive interpolation may be applied due to lack of EA data points. Moreover, there is no general method to estimate the overall EA quality of a map. In particular, there is no indication, per map region, that can easily reflect the EA quality of the output of the EA algorithms used in analyzing and visualizing the EA map therein.

Examples of the present disclosure that are described herein provide a technique comprising an automated algorithm that provides an overlapping map that scores, at each location on the EA map, the EA quality of the output of the one or more EA algorithms used to construct the EA map. The disclosed technique can guide the physician to avoid misinterpretation of an EA map. Rather than accept an EA map as is, based on the indication from the disclosed method, the physician may try to improve an insufficient EA analysis by remapping, or by correcting the analysis, of specific cardiac chamber areas.

When using the LCV algorithm, a map region may receive a low score due to, for example, areas where conduction vectors are purposely omitted by the algorithm due to the fact there is no mutual trend in LAT. A physician, unaware of that intentional omission, may miss out on clinically important fractionated areas that due to the nature of the conduction through a sick tissue do not present a strong LAT trend but rather a circular multidirectional propagation. By shading an area with low EA algorithms outputs quality in a map, the disclosed technique alerts the physician against making such an error.

Another example of a map region with a low quality score is an area in a coherent EA map where excessive interpolations have been made. A physician, unaware of the excessive interpolation, trusts the EA map without suspecting that actual data in that area is sparse. Again, by shading an area with low EA quality in a map, the disclosed technique alerts the physician against making such an error.

In one example, a method is provided that includes receiving an EA-mapped portion of a cardiac chamber. The portion includes analyzed EA properties of the cardiac chamber using one or more EA analysis algorithms. A processor runs an algorithm that scores the quality of the EA analysis output over the EA mapped portion. In an example, the EA quality score may be normalized to a range between 0 and 1, and, based on this score, the processor estimates if the EA quality is insufficient (e.g., failing to meet EA quality a defined quality criterion) in one or more regions of the EA mapped portion. The processor marks any EA that is found insufficient in one or more regions on an EA map. The indicated map is displayed to a user.

The processor may indicate such an insufficient EA analyzed map area by building a gray scale map of locations only above a high threshold or below a low threshold. The processor may blend (e.g., combine) the gray scale information with current EA map coloring. The blending can be reflected in several visualizations, such as applying shading (e.g., a lower level of transparency) to an area having a quality score below a given threshold. In another blending example, the processor overlays the EA map with a layer of graphical symbols that vary in format according to the quality score.

The inputs for a scoring algorithm are dependent on the clinical problem. Different clinical scenarios (e.g., atrial vs ventricular arrhythmias) use different EA algorithms. The disclosed technique estimates the algorithm output quality in order to score the relevant EA map. In some examples, the EA algorithm is preselected, and the map is received and estimated as is. In other examples, a user can select or unselect all or part of the above EA analysis algorithms (or others) via a graphical user interface (GUI). The selection may be available, for example, as check boxes in the GUI.

In an example elaborated below, the quality score is a weighted sum of quality scores of outputs from two or more EA analysis algorithms (for example of bipolar amplitudes and TPI values).

In another example, to indicate insufficiency of EA analysis in one or more regions on an EA map, the disclosed technique may apply the steps of filtering outlier data points (based on the score), projecting data points to the anatomical map surface, and then applying Laplacian interpolation to the quality score.

SYSTEM DESCRIPTION

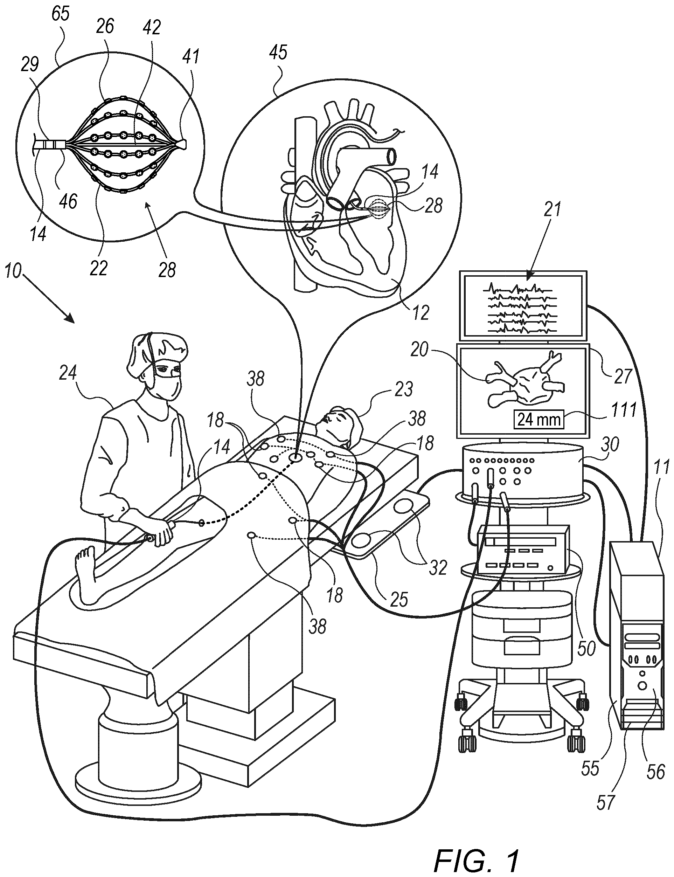

Fig. 1 is a schematic, pictorial illustration of a catheter-based electroanatomical (EA) mapping and ablation system 10, in accordance with an example of the present disclosure.

System 10 includes multiple catheters which are percutaneously inserted by physician 24 through the patient's vascular system into a chamber or vascular structure of a heart 12 (seen in inset 45). Typically, a delivery sheath catheter is inserted into a cardiac chamber, such as the left or right atrium near a desired location in heart 12. Thereafter, a plurality of catheters can be inserted into the delivery sheath catheter so as to arrive at the desired location. The plurality of catheters may include a catheter dedicated for pacing, a catheter for sensing intracardiac electrogram signals, a catheter dedicated for ablating and/or a catheter dedicated for both EA mapping and ablating. An example catheter 14, illustrated herein, is configured for sensing bipolar electrograms. Physician 24 brings a distal tip 28 (also called hereinafter distal end assembly 28) of catheter 14 into contact with the heart wall for sensing a target site in heart 12. For ablation, physician 24 similarly brings a distal end of an ablation catheter to a target site.

As seen in inset 65, catheter 14 is an exemplary catheter that includes a basket distal end 28, including one, and preferably multiple, electrodes 26 optionally distributed over a plurality of splines 22 at distal tip 28 and configured to sense IEGM signals. Catheter 14 may additionally include a position sensor 29 embedded in or near distal tip 28 on a shaft 46 of catheter 14, for tracking the position and orientation of distal tip 28. Optionally, and preferably, position sensor 29 is a magnetic-based position sensor including three magnetic coils for sensing three-dimensional (3D) position and orientation. As seen, distal tip 28 further includes an expansion/collapse rod 42 of expandable assembly 28 that is mechanically connected to basket assembly 28 at a distal edge 41 of assembly 28.

Magnetic based position sensor 29 may be operated together with a location pad 25 that includes a plurality of magnetic coils 32 configured to generate magnetic fields in a predefined working volume. Real-time position of distal tip 28 of catheter 14 may be tracked based on magnetic fields generated with location pad 25 and sensed by magnetic based position sensor 29. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish a location reference for location pad 25 as well as impedance-based tracking of electrodes 26. For impedance-based tracking, electrical current is directed toward electrodes 26 and sensed at electrode skin patches 38 so that the location of each electrode can be triangulated via electrode patches 38. Details of the impedance-based location tracking technology are described in US Patent Nos. 7,536,218; 7,756,576; 7,848,787; 7,869,865; and 8,456,182.

A recorder 11 displays cardiac signals 21 (e.g., electrograms acquired at respectively tracked cardiac tissue positions) acquired with body surface ECG electrodes 18 and intracardiac electrograms acquired with electrodes 26 of catheter 14. Recorder 11 may include pacing capability to pace the heart rhythm, and/or may be electrically connected to a standalone pacer.

System 10 may include an ablation energy generator 50 that is adapted to conduct ablative energy to one or more electrodes at a distal tip of a catheter configured for ablation. Energy produced by ablation energy generator 50 may include, but is not limited to, radiofrequency (RF) energy or pulsed-field ablation (PFA) energy, including monopolar or bipolar high-voltage DC pulses, to be used to effect irreversible electroporation (IRE), or combinations thereof.

Patient interface unit (PIU) 30 is an interface configured to establish electrical communication between catheters, electrophysiological equipment, power supply and a workstation 55 to control system 10 operation and to receive EA signals from the catheter. Electrophysiological equipment of system 10 may include, for example, multiple catheters, location pad 25, body surface ECG electrodes 18, electrode patches 38, ablation energy generator 50, and recorder 11. Optionally, and preferably, PIU 30 additionally includes processing capability for implementing real-time computations of catheter locations and for performing ECG calculations. Interface 30 may also be configured to receive a file (e.g., a file of an EA map) using, for exmaple, a communication line or a memory stick.

Workstation 55 includes memory 57, a processor 56 unit with memory or storage with appropriate operating software loaded therein, and user interface capability. Workstation 55 may provide multiple functions, optionally including (i) modeling endocardial anatomy in three-dimensions (3D) and rendering the model or anatomical map 20 for display on a display device 27, (ii) displaying on display device 27 activation sequences (or other data) compiled from recorded cardiac signals 21 in representative visual indicia or imagery superimposed on the rendered anatomical map 20, (iii) displaying real-time location and orientation of multiple catheters within the heart chamber, and (iv) displaying sites of interest on display device 27 such as places where ablation energy has been applied. One commercial product embodying elements of system 10 is available as the CARTO™ 3 System, available from Biosense Webster, Inc., 31A Technology Drive, Irvine, CA 92618.

In a disclosed example, processor 56 runs an algorithm that provides an overlapping map (e.g., comprising a shading layer superimposed on anatomical map 20) that represents a score of the EA quality of the EA map at each location on the EA map. The EA analysis algorithms may be preselected, or physician 24 can select/unselect, e.g., via a GUI 111, at least a portion of the EA analysis algorithms listed above to be used in the EA map analysis.

In some examples, processor 56 typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

This configuration of system 10 is shown by way of example, in order to illustrate certain problems that are addressed by examples of the present disclosure and to demonstrate the application of these examples in enhancing the performance of such a system. Examples of the present disclosure, however, are by no means limited to this specific sort of example system, and the principles described herein may similarly be applied to other types of medical systems. For example, other multi-electrode catheter types may be used, such as the OCTARAY™ catheter or a flat catheter.

VISUALIZATION OF EA ANALYSIS QUALITY OF AN EA MAP OF A CARDIAC CHAMBER

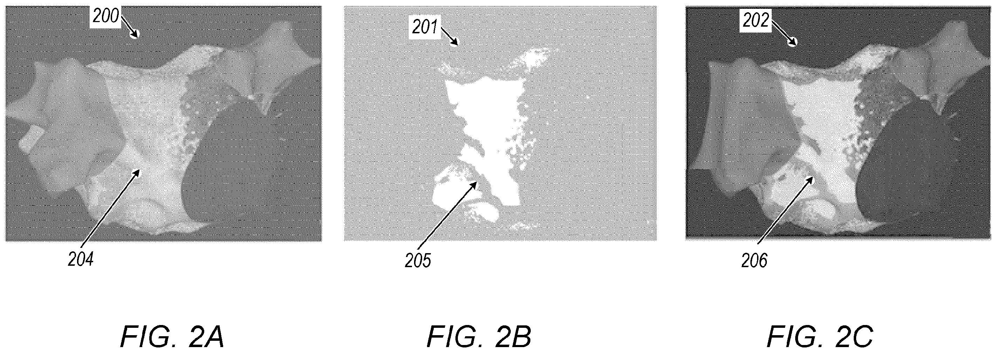

Figs. 2A-2C are, as follows: Fig. 2A is an EA map of a left atrium; Fig. 2B is a shading mask to indicate regions of the EA map with insufficient EA analysis quality; Fig. 2C is a combined EA map that results from overlaying the mask of Fig. 2B over the EA map of Fig. 2A, in accordance with an example of the present disclosure.

EA map 200 (seen in Fig. 2A) comprises an anatomical surface and respective electrical values (e.g., activation amplitudes and/or times) at each mapped location over the surface. Interpolation over EA values is used in an EA map in order to provide an EA map of continuous format. However, in some regions of EA map 200 the interpolation may be of low EA analysis quality, such as in region 204.

The disclosed algorithm scores the EA analysis quality and provides the score in a form of shading mask 201 (seen in Fig. 2B). Shading mask 201 comprises a quality score for at least some of the regions of EA map 200, the score normalized in the range [0,1].

As seen in Fig. 2B, shading mask 201 includes a dark region 205 where the score is low. The dark areas indicate to the user that the EA analysis quality in these regions is insufficient, and therefore unreliable.

Combined EA map 202 (seen in Fig. 2C) shows this low-quality EA analysis as a gray region 206. Based on this indication, the user may, for example, EA remap region 206.

More generally, blending the information from a quality mask with EA map 200 coloring can be reflected by several visualizations:

1. As a transparent layer, where the transparency and a gray level color is also affected by the score.
2. Bump mapping, where the bump is affected by the score (e.g., high score indicating a small bump, a low score indicating a bigger bump).

In an example implementation, a processor receives a region of interest (ROI) on an anatomical map.

The quality is computed from EA analysis algorithms based on the criteria below:

- LCV incoherency (possible indication of fractionated signals)
- Data point tag distance from surface (geometric anatomical inaccuracies)
- Contact level TPI
- Density of data points
- Coherent areas of excessive interpolation

All or a portion of the above algorithms (or others) can be preselected, or selected/unselected by the user via GUI 111 of Fig. 1.

Finally, the score visualization seen in the example of Fig. 2B is of EA analysis quality of an atrial fibrillation (AFib) map, but, in general, the score is problem-dependent. Different clinical scenarios (e.g., atrial vs ventricular arrhythmias) use different EA analysis algorithms due to the disclosed technique use of their outputs to score the relevant EA map.

SCORING OF EA ANALYSIS QUALITY OF AN EA MAP OF A CARDIAC CHAMBER

An example of the disclosed method of calculating an EA quality score is provided herein. Assuming features $F_i$ of selected respective EA analysis algorithms (e.g., bipolar amplitude and TPI):

- Each $F_i$ has a range of $m_i < F_i < M_i$
- At each location $p$ on the map there is a value for each feature $F_i(p)$

An EA quality score can then be given by the weighted sum

$$Score(p) = \sum_i w_i \varphi_i \left( \frac{F_i(p)}{M_i - m_i} \right), \quad \left( 0 \le w_i \le 1, \ \sum_i w_i = 1 \right),$$

where $\varphi_i$ is any spanning basis function set.

METHOD OF ESTIMATION AND VISUALIZATION OF EA ANALYSIS QUALITY OF AN EA MAP OF A CARDIAC CHAMBER

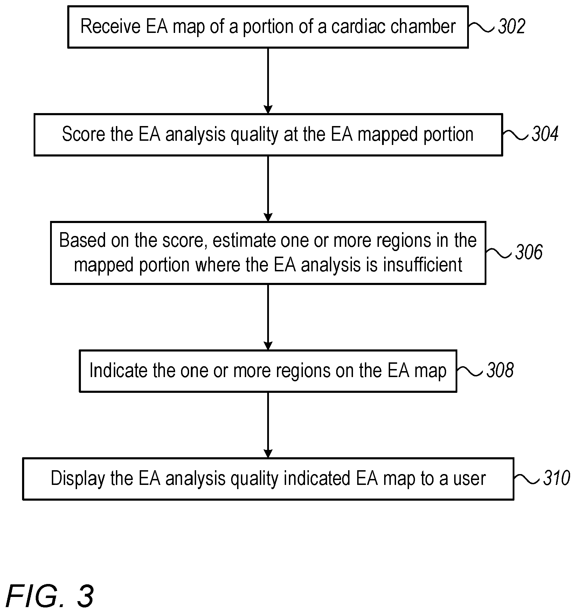

Fig. 3 is a flow chart that schematically illustrates a method of estimation and visualization of quality of an EA map 200, in accordance with an example of the present disclosure. The algorithm, according to the presented example, carries out a process that begins at an EA map receiving step 302, with processor 56 receiving an EA map that was EA analyzed using an EA analysis algorithm (such as the aforementioned LCV). Note that a physician 24 may adjust the EA analysis parameters or algorithms used via GUI 111.

Next, the processor runs a disclosed algorithm that gives EA quality scores to the EA analysis at the EA map, at scoring step 304.

Based on the scores, the processor estimates one or more regions of the EA map (e.g., of EA map 200) as having too low (e.g., insufficient) EA analysis quality, at region estimation step 306.

At area indication step 308, the processor indicates (e.g., marks) one or more areas (e.g., regions) of the EA map as having insufficient EA analysis quality (e.g., failing to meet EA quality a defined quality criterion).

Finally, at an EA quality indicated map presenting step 310, processor 56 presents to a user the EA quality indicated EA map (e.g., EA map 202).

The flow chart shown in Fig. 3 is chosen purely for the sake of conceptual clarity. Other examples of the technique may comprise different algorithmic and/or visualization steps, such as using blinking to highlight areas scored by the disclosed technique as possessing insufficient EA analysis quality.

EXAMPLES

Example 1

A method includes receiving an electroanatomical (EA) map (200) of at least a portion of a cardiac chamber. A quality is scored of an EA analysis of the EA map. Based on the scoring, one or more regions (204) are identified in the mapped portion as failing to meet a defined quality criterion. The one or more regions are indicted (206) on an EA map (202) to be displayed to a user.

Example 2

The method according to example 1, wherein the EA analysis is performed by at least one algorithm selected from a list of algorithms consisting of (i) Local Conduction Vectors (LCV), (ii) Coherent EA (CEA) propagation, (iii) Touch Indication Index (TPI), (iv) Late Activation times (LAT), and (v) Fractionation Score.

Example 3

The method according to any of examples 1 and 2, wherein scoring the quality includes calculating a weighted sum of scores of qualities of outputs from two or more algorithms from the list.

Example 4

The method according to any of examples 1 through 3, wherein indicating (206) the one or more regions on the EA map comprises overlaying the EA map with a layer of transparency according to the scoring.

Example 5

The method according to any of examples 1 through 4, wherein indicating (206) the one or more regions on the EA map comprises overlaying the EA map with a layer of graphical symbols that vary in format according to the scoring.

Example 6

The method according to any of examples 1 through 5, wherein indicating (206) the one or more regions (204) on the EA map comprises (i) filtering outlier points based on the scoring, (ii) projecting the scoring onto a surface of the map, and (iii) applying Laplacian interpolation to the scoring.

Example 7

The method according to any of examples 1 through 6, and comprising displaying the EA map (202), including the identified one or more regions (204), to a user.

Example 8

A system (10) includes an interface (30) and a processor (56). The interface is configured to receive an electroanatomical (EA) map (200) of at least a portion of a cardiac chamber. The processor (56) is configured to (i) score a quality of an EA analysis of the EA map, (ii) based on the scoring, identify one or more regions (204) in the mapped portion as failing to meet a defined quality criterion, and (iii) indicate (206) the one or more regions on an EA map (202) to be displayed to a user.

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as

**EP 4 494 561 A1**

variations and modifications thereof which would occur to persons skilled in the art upon reading the foregoing description and which are not disclosed in the prior art.

**Claims**

1. A method, comprising:

   receiving an electroanatomical (EA) map (200) of at least a portion of a cardiac chamber;
   scoring a quality of an EA analysis of the EA map;
   based on the scoring, identifying one or more regions (204) in the mapped portion as failing to meet a defined quality criterion; and
   indicating (206) the one or more regions on an EA map (202) to be displayed to a user.

2. The method according to claim 1, wherein the EA analysis is performed by at least one algorithm selected from a list of algorithms consisting of (i) Local Conduction Vectors (LCV), (ii) Coherent EA (CEA) propagation, (iii) Touch Indication Index (TPI), (iv) Late Activation times (LAT), and (v) Fractionation Score.

3. The method according to claim 1 or claim 2, wherein scoring the quality comprises calculating a weighted sum of scores of qualities of outputs from two or more algorithms from the list.

4. The method according to any preceding claim, wherein indicating (206) the one or more regions (204) on the EA map comprises overlaying the EA map with a layer of transparency according to the scoring.

5. The method according to any preceding claim, wherein indicating (206) the one or more regions (204) on the EA map comprises overlaying the EA map with a layer of graphical symbols that vary in format according to the scoring.

6. The method according to any preceding claim, wherein indicating (206) the one or more regions (204) on the EA map comprises (i) filtering outlier points based on the scoring, (ii) projecting the scoring onto a surface of the map, and (iii) applying Laplacian interpolation to the scoring.

7. The method according to any preceding claim, and comprising displaying the EA map (202), including the identified one or more regions (204), to a user.

8. A system (10), comprising:

   an interface (30) configured to receive an electroanatomical (EA) map (200) of at least a portion of a cardiac chamber; and
   a processor (56), which is configured to:

      score a quality of an EA analysis of the EA map;
      based on the scoring, identify one or more regions (204) in the mapped portion as failing to meet a defined quality criterion; and
      indicate (206) the one or more regions on an EA map (202) to be displayed to a user.

9. The system according to claim 8, wherein the EA analysis is performed by at least one algorithm selected from a list of algorithms consisting of (i) Local Conduction Vectors (LCV), (ii) Coherent EA (CEA) propagation, (iii) Touch Indication Index (TPI), (iv) Late Activation times (LAT), and (v) Fractionation Score.

10. The system according to claim 8 or 9, wherein the processor is configured to score the quality by calculating a weighted sum of scores of qualities of outputs from two or more algorithms from the list.

11. The system according to any of claims 8 to 10, wherein the processor is configured to indicate (206) the one or more regions (204) on the EA map by overlaying the EA map with a layer of transparency according to the scoring.

12. The system according to any of claims 8 to 11, wherein the processor is configured to indicate (206) the one or more regions (204) on the EA map by overlaying the EA map with a layer of graphical symbols that vary in format according to the scoring.

7

13. The system according to any of claims 8 to 12, wherein the processor is configured to indicate (206) the one or more regions (204) on the EA map by (i) filtering outlier points based on the scoring, (ii) projecting the scoring onto a surface of the map, and (iii) applying Laplacian interpolation to the scoring.

14. The system according to any of claims 8 to 13, wherein the processor is further configured to display the EA map (202), including the identified one or more regions 9204), to a user.

**FIG. 1**

*FIG. 2A*

*FIG. 2B*

*FIG. 2C*

Receive EA map of a portion of a cardiac chamber — 302

Score the EA analysis quality at the EA mapped portion — 304

Based on the score, estimate one or more regions in the mapped portion where the EA analysis is insufficient — 306

Indicate the one or more regions on the EA map — 308

Display the EA analysis quality indicated EA map to a user — 310

*FIG. 3*

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/211314 A1 (GOVARI ASSAF [IL] ET AL) 7 July 2022 (2022-07-07)<br>* paragraphs [0038] - [0048], [0050] - [0051], [0066] - [0069] *<br>* figures 2, 4A-4B * | 1-14 | INV.<br>A61B5/367<br>A61B5/00 |
| X | US 2022/095942 A1 (GOVARI ASSAF [IL] ET AL) 31 March 2022 (2022-03-31)<br>* paragraphs [0037] - [0038], [0042] - [0048] * | 1-14 | |

**TECHNICAL FIELDS SEARCHED (IPC)**

A61B

The present search report has been drawn up for all claims

| Place of search | Date of completion of the search | Examiner |
|---|---|---|
| The Hague | 12 November 2024 | Faymann, Juan |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons

.......................................................................................

& : member of the same patent family, corresponding
 document

EPO FORM 1503 03.82 (P04C01)

## ANNEX TO THE EUROPEAN SEARCH REPORT
## ON EUROPEAN PATENT APPLICATION NO.

EP 24 18 9619

This annex lists the patent family members relating to the patent documents cited in the above-mentioned European search report.
The members are as contained in the European Patent Office EDP file on
The European Patent Office is in no way liable for these particulars which are merely given for the purpose of information.

12-11-2024

| Patent document cited in search report | Publication date | Patent family member(s) | | Publication date |
|---|---|---|---|---|
| US 2022211314 A1 | 07-07-2022 | CN | 114711779 A | 08-07-2022 |
| | | EP | 4026497 A1 | 13-07-2022 |
| | | IL | 289283 A | 01-08-2022 |
| | | JP | 2022106683 A | 20-07-2022 |
| | | US | 2022211314 A1 | 07-07-2022 |
| US 2022095942 A1 | 31-03-2022 | CN | 114305448 A | 12-04-2022 |
| | | EP | 3973876 A1 | 30-03-2022 |
| | | IL | 286525 A | 01-04-2022 |
| | | JP | 2022056404 A | 08-04-2022 |
| | | US | 2022095942 A1 | 31-03-2022 |

For more details about this annex : see Official Journal of the European Patent Office, No. 12/82

**EP 4 494 561 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 11478182 B
- US 55391199 B
- US 5443489 A
- US 5558091 A
- US 6172499 B
- US 6239724 B
- US 6332089 B
- US 6484118 B
- US 6618612 B
- US 6690963 B
- US 6788967 B
- US 6892091 B
- US 7536218 B
- US 7756576 B
- US 7848787 B
- US 7869865 B
- US 8456182 B